# EUROPEAN PATENT SPECIFICATION

(11) **EP 1 504 125 B1**
(45) Date of publication and mention of the grant of the patent: **29.10.2008**
(21) Application number: 03730032.4
(22) Date of filing: 14.05.2003
(51) Int. Cl.: C12Q 1/68

(54) **HUMAN OBESITY SUSCEPTIBILITY GENE AND USES THEREOF**
HUMANES FETTLEIBIGKEIT SUSZEPTIBILITÄTSGEN UND DEREN VERWENDUNGEN
GENE HUMAIN DE PREDISPOSITION A L'OBESITE ET UTILISATIONS DUDIT GENE

(30) Priority: 15.05.2002 EP 02291201; 27.09.2002 EP 02292392
(43) Date of publication of application: 09.02.2005
(60) Divisional application: 08159663.7
(73) Proprietor: Integragen, 91000 Evry (FR)
(72) Inventor: BROOKS, Peter, F-91000 Evry (FR); ROSCHMANN, Elke, D - 89179 Bremerstetten (DE); PHILIPPI, Anne, F-77310 St. Fargeau Ponthierry (FR); HAGER, Jörg, F-91540 Mennecy (FR)
(74) Representative: Gallois, Valérie
(86) International application number: PCT/EP2003/005052
(87) International publication number: WO 2003/097683

(56) References cited:
- WO-A-00/30674
- WO-A-01/18250
- WO-A-95/17906
- WO-A-96/14331
- SAINSBURRY AMANDA ET AL: "Y4 receptor knockout rescues fertility in ob/ob mice." GENES & DEVELOPMENT, vol. 16, no. 9, 1 May 2002 (2002-05-01), pages 1077-1088, XP002229231 May 1, 2002 ISSN: 0890-9369
- PARKER ERIC ET AL: "Neuropeptide Y receptors as targets for anti-obesity drug development: Perspective and current status." EUROPEAN JOURNAL OF PHARMACOLOGY, vol. 440, no. 2-3, 2002, pages 173-187, XP002229232 12 April, 2002 ISSN: 0014-2999
- DATABASE EMBL [Online] 17 May 1996 (1996-05-17), YAN ET AL.: "Rattus norvegicus pancreatic polypeptide receptor mRNA, complete CDS" XP002250172 Database accession no. U42388
- DATABASE EMBL [Online] 14 October 2000 (2000-10-14), LEWIN ET AL.: "Bovine 5' EST" XP002250173 Database accession no. BF041396
- BARD JONATHAN A ET AL: "Cloning and functional expression of a human Y4 subtype receptor for pancreatic polypeptide, neuropeptide Y, and peptide YY." JOURNAL OF BIOLOGICAL CHEMISTRY, vol. 270, no. 45, 1995, pages 26762-26765, XP002187958 ISSN: 0021-9258
- DATABASE NCBI-SNP [Online] NCBI SNP Database 29 August 2001 (2001-08-29), XP002229234 accession no. HTTP://WWW.NCBI.NLM.NIH.GOV:80/ENTREZ/QUER Y.FCGI?D Database accession no. rs2229967
- DATABASE JSNP [Online] Japanese SNP Database 17 July 2002 (2002-07-17), "SNP Information for IMS-JST185880" XP002229235 accession no. HTTP://SNP.IMS.U-TOKYO.AC.JP/ Database accession no. IMS-JST185880
- NELSON STANLEY F ET AL: "Genomic mismatch scannings: A new approach to genetic linkage mapping." NATURE GENETICS, vol. 4, no. 1, 1993, pages 11-18, XP000606264 ISSN: 1061-4036
- BROOKS P ET AL: "Genomic hybrid identity profiling (GenomeHIP) of chromosomal regions identical by descent." AMERICAN JOURNAL OF HUMAN GENETICS, vol. 71, no. 4 Supplement, October 2002 (2002-10), page 408, XP001109541 52nd Annual Meeting of the American Society of Human Genetics;Baltimore, MD, USA; October 15-19, 2002, October, 2002 ISSN: 0002-9297

### SEQUENCE LISTING

<110> Integragen
<120> Human obesity susceptibility gene and uses thereof
<130> BO138WO
<140>
   <141>
<150> EP 02 291201.8
   <151> 2002-05-15
<150> EP 02 292392.4
   <151> 2002-09-27
<160> 10
<170> PatentIn Ver. 2.1
<210> 1
   <211> 1673
   <212> DNA
   <213> Homo sapiens
<220>
   <221> CDS
   <222> (86)..(1213)
<400> 1
   ttaataatga ctagagaatc tgagaggcgt catccctcaa gtgtatcact tagttcaaga 60
   gtcctggaat cttttcacat ccact atg aac acc tct cac ctc ctg gcc ttg   112
                               Met Asn Thr Ser His Leu Leu Ala Leu
                               1               5
   ctg ctc cca aaa tct cca caa ggt gaa aac aga agc aaa ccc ctg ggc   160
   Leu Leu Pro Lys Ser Pro Gln Gly Glu Asn Arg Ser Lys Pro Leu Gly
   10                  15                  20                  25
   acc cca tac aac ttc tct gaa cat tgc cag gat tcc gtg gac gtg atg   208
   Thr Pro Tyr Asn Phe Ser Glu His Cys Gln Asp Ser Val Asp Val Met
                   30                  35                  40
   gtc ttc atc gtc act tcc tac agc att gag act gtc gtg ggg gtc ctg   256
   Val Phe Ile Val Thr Ser Tyr Ser Ile Glu Thr Val Val Gly Val Leu
               45                  50                  55
   ggt aac ctc tgc ctg atg tgt gtg act gtg agg cag aag gag aaa gcc   304
   Gly Asn Leu Cys Leu Met Cys Val Thr Val Arg Gln Lys Glu Lys Ala
           60                  65                  70
   aac gtg acc aac ctg ctt atc gcc aac ctg gcc ttc tct gac ttc ctc   352
   Asn Val Thr Asn Leu Leu Ile Ala Asn Leu Ala Phe Ser Asp Phe Leu
       75                  80                  85
   atg tgc ctc ctc tgc cag ccg ctg acc gcc gtc tac acc atc atg gac   400
   Met Cys Leu Leu Cys Gln Pro Leu Thr Ala Val Tyr Thr Ile Met Asp
   90                  95                  100                 105
   tac tgg atc ttt gga gag acc ctc tgc aag atg tcg gcc ttc atc cag     448
   Tyr Trp Ile Phe Gly Glu Thr Leu Cys Lys Met Ser Ala Phe Ile Gln
                   110                 115                 120
   tgc atg tcg gtg acg gtc tcc atc ctc tcg ctc gtc ctc gtg gcc ctg     496
   Cys Met Ser Val Thr Val Ser Ile Leu Ser Leu Val Leu Val Ala Leu
               125                 130                 135
   gag agg cat cag ctc atc atc aac cca aca ggc tgg aag ccc agc atc     544
   Glu Arg His Gln Leu Ile Ile Asn Pro Thr Gly Trp Lys Pro Ser Ile
           140                 145                 150
   tca cag gcc tac ctg ggg att gtg ctc atc tgg gtc att gcc tgt gtc     592
   Ser Gln Ala Tyr Leu Gly Ile Val Leu Ile Trp Val Ile Ala Cys Val
       155                 160                 165
   ctc tcc ctg ccc ttc ctg gcc aac agc atc ctg gag aat gtc ttc cac     640
   Leu Ser Leu Pro Phe Leu Ala Asn Ser Ile Leu Glu Asn Val Phe His
   170                 175                 180                 185
   aag aac cac tcc aag gct ctg gag ttc ctg gcg gat aag gtg gtc tgt     688
   Lys Asn His Ser Lys Ala Leu Glu Phe Leu Ala Asp Lys Val Val Cys
                   190                 195                 200
   acc gag tcc tgg cca ctg gct cac cac cgc acc atc tac acc acc ttc     736
   Thr Glu Ser Trp Pro Leu Ala His His Arg Thr Ile Tyr Thr Thr Phe
               205                 210                 215
   ctg ctc ctc ttc cag tac tgc ctc cca ctg ggc ttc atc ctg gtc tgt     784
   Leu Leu Leu Phe Gln Tyr Cys Leu Pro Leu Gly Phe Ile Leu Val Cys
           220                 225                 230
   tat gca cgc atc tac cgg cgc ctg cag agg cag ggg cgc gtg ttt cac     832
   Tyr Ala Arg Ile Tyr Arg Arg Leu Gln Arg Gln Gly Arg Val Phe His
       235                 240                 245
   aag ggc acc tac agc ttg cga gct ggg cac atg aag cag gtc aat gtg     880
   Lys Gly Thr Tyr Ser Leu Arg Ala Gly His Met Lys Gln Val Asn Val
   250                 255                 260                 265
   gtg ctg gtg gtg atg gtg gtg gcc ttt gcc gtg ctc tgg ctg cct ctg     928
   Val Leu Val Val Met Val Val Ala Phe Ala Val Leu Trp Leu Pro Leu
                   270                 275                 280
   cat gtg ttc aac agc ctg gaa gac tgg cac cat gag gcc atc ccc atc     976
   His Val Phe Asn Ser Leu Glu Asp Trp His His Glu Ala Ile Pro Ile
               285                 290                 295
   tgc cac ggg aac ctc atc ttc tta gtg tgc cac ttg ctt gcc atg gcc     1024
   Cys His Gly Asn Leu Ile Phe Leu Val Cys His Leu Leu Ala Met Ala
           300                 305                 310
   tcc acc tgc gtc aac cca ttc atc tat ggc ttt ctc aac acc aac ttc     1072
   Ser Thr Cys Val Asn Pro Phe Ile Tyr Gly Phe Leu Asn Thr Asn Phe
       315                 320                 325
   aag aag gag atc aag gcc ctg gtg ctg act tgc cag cag agc gcc ccc     1120
   Lys Lys Glu Ile Lys Ala Leu Val Leu Thr Cys Gln Gln Ser Ala Pro
   330                 335                 340                 345
   ctg gag gag tcg gag cat ctg ccc ctg tcc aca gta cat acg gaa gtc     1168
   Leu Glu Glu Ser Glu His Leu Pro Leu Ser Thr Val His Thr Glu Val
                   350                 355                 360
   tcc aaa ggg tcc ctg agg cta agt ggc agg tcc aat ccc att taa       1213
   Ser Lys Gly Ser Leu Arg Leu Ser Gly Arg Ser Asn Pro Ile
               365                 370                 375
   ccaggtctag gtcttctccc tgccatgtcc cttgccaggc tcttccactt agctaagtgg 1273
   gcacactgca agctggggtg gcaccccagc attcctggct ttctggggtc cagataggct 1333
   ggcaagagct gtttttgcat ccatttgcat cgtgaagact ggcattttga tacttcagct 1393
   gtttgttcct gggagaattc tgagcacaga ttccagaggt cacagtaagc cttgcagctt 1453
   gagctgaaag atgccagagc cggagatgtc tgctggcagc aggcagggtt cattctggtg 1513
   acacagcaac agatgcctgg cctgggaacc cagggatttc acctccacca gtgagaccac 1573
   ggggccactg tggggtgagg gaaggagcgc ttggagtcag agctctagag tcgacctgca 1633
   ggcatgcaag cttggcactg aagccgaatt ccagcacact                       1673
<210> 2
   <211> 375
   <212> PRT
   <213> Homo sapiens
<400> 2
   Met Asn Thr Ser His Leu Leu Ala Leu Leu Leu Pro Lys Ser Pro Gln
   1 5 10 15
   Gly Glu Asn Arg Ser Lys Pro Leu Gly Thr Pro Tyr Asn Phe Ser Glu
   20 25 30
   His Cys Gln Asp Ser Val Asp Val Met Val Phe Ile Val Thr Ser Tyr
   35 40 45
   Ser Ile Glu Thr Val Val Gly Val Leu Gly Asn Leu Cys Leu Met Cys
   50 55 60
   Val Thr Val Arg Gln Lys Glu Lys Ala Asn Val Thr Asn Leu Leu Ile
   65 70 75 80
   Ala Asn Leu Ala Phe Ser Asp Phe Leu Met Cys Leu Leu Cys Gln Pro
   85 90 95
   Leu Thr Ala Val Tyr Thr Ile Met Asp Tyr Trp Ile Phe Gly Glu Thr
   100 105 110
   Leu Cys Lys Met Ser Ala Phe Ile Gln Cys Met Ser Val Thr Val Ser
   115 120 125
   Ile Leu Ser Leu Val Leu Val Ala Leu Glu Arg His Gln Leu Ile Ile
   130 135 140
   Asn Pro Thr Gly Trp Lys Pro Ser Ile Ser Gln Ala Tyr Leu Gly Ile
   145 150 155 160
   Val Leu Ile Trp Val Ile Ala Cys Val Leu Ser Leu Pro Phe Leu Ala
   165 170 175
   Asn Ser Ile Leu Glu Asn Val Phe His Lys Asn His Ser Lys Ala Leu
   180 185 190
   Glu Phe Leu Ala Asp Lys Val Val Cys Thr Glu Ser Trp Pro Leu Ala
   195 200 205
   His His Arg Thr Ile Tyr Thr Thr Phe Leu Leu Leu Phe Gln Tyr Cys
   210 215 220
   Leu Pro Leu Gly Phe Ile Leu Val Cys Tyr Ala Arg Ile Tyr Arg Arg
   225 230 235 240
   Leu Gln Arg Gln Gly Arg Val Phe His Lys Gly Thr Tyr Ser Leu Arg
   245 250 255
   Ala Gly His Met Lys Gln Val Asn Val Val Leu Val Val Met Val Val
   260 265 270
   Ala Phe Ala Val Leu Trp Leu Pro Leu His Val Phe Asn Ser Leu Glu
   275 280 285
   Asp Trp His His Glu Ala Ile Pro Ile Cys His Gly Asn Leu Ile Phe
   290 295 300
   Leu Val Cys His Leu Leu Ala Met Ala Ser Thr Cys Val Asn Pro Phe
   305 310 315 320
   Ile Tyr Gly Phe Leu Asn Thr Asn Phe Lys Lys Glu Ile Lys Ala Leu
   325 330 335
   Val Leu Thr Cys Gln Gln Ser Ala Pro Leu Glu Glu Ser Glu His Leu
   340 345 350
   Pro Leu Ser Thr Val His Thr Glu Val Ser Lys Gly Ser Leu Arg Leu
   355 360 365
   Ser Gly Arg Ser Asn Pro Ile
   370 375
<210> 3
   <211> 19
   <212> DNA
   <213> Artificial sequence
<220>
   <223> primer
<400> 3
   taagtggaag agcctggca 19
<210> 4
   <211> 21
   <212> DNA
   <213> artificial sequence
<220>
   <223> primer
<400> 4
   cctggagaat gtcttccaca a 21
<210> 5
   <211> 19
   <212> DNA
   <213> artificial sequence
<220>
   <223> primer
<400> 5
   aggtggtgta gatggtgcg 19
<210> 6
   <211> 21
   <212> DNA
   <213> artificial sequence
<220>
   <223> primer
<400> 6
   ttctctgaac attgccagga t 21
<210> 7
   <211> 21
   <212> DNA
   <213> artificial sequence
<220>
   <223> primer
<400> 7
   agtccatgat ggtgtagacg g 21
<210> 8
   <211> 22
   <212> DNA
   <213> artificial sequence
<220>
   <223> primer
<400> 8
   agcatgcaac aagtgcttct aa 22
<210> 9
   <211> 20
   <212> DNA
   <213> artificial sequence
<220>
   <223> primer
<400> 9
   cagggctcta gcacatgaat 20
<210> 10
   <211> 20
   <212> DNA
   <213> artificial sequence
<220>
   <223> primer
<400> 10
   cagggctcta gcacatgaac 20

## Description

FIELD OF THE INVENTION

The present invention relates generally to the fields of genetics and medicine. The present invention more particularly discloses the identification of a human obesity susceptibility gene, which can be used for the diagnosis of obesity. The invention more specifically discloses certain alleles of the pancreatic polypeptide receptor 1 (PPYR1, *NPY4R)* gene related to susceptibility to obesity and representing novel targets for therapeutic intervention. The present invention relates to particular mutations in the PPYR1 gene. The invention can be used in the diagnosis of predisposition to, obesity.

### BACKGROUND OF THE INVENTION

Approximately three to eight percent of the total health costs of modem industrialized countries are currently due to the direct costs of obesity (Wolf, 1996). In Germany, the total costs (both direct and indirect) related to obesity and comorbid disorders were estimated at 21 billion German marks in 1995 (Schneider, 1996). By 2030 these costs will rise by 50% even if the prevalence of obesity does not increase further.

Obesity is often defined simply as a condition of abnormal or excessive fat accumulation in adipose tissue, to the extent that health may be impaired. The underlying disease is the process of undesirable positive energy balance and weight gain. An abdominal fat distribution is associated with higher health risks than a gynoid fat distribution.

The body mass index (BMI; kg/m²) provides the most useful, albeit crude, population-level measure of obesity. It can be used to estimate the prevalence of obesity within a population and the risks associated with it. However, BMI does not account for body compositon or body fat distribution (WHO, 1998).

**Table 1: Classification of overweight in adults according to BMI (WHO, 1998)**

| Classification | BMI (kg/m²) | Risk of co-morbidities |
|---|---|---|
| Underweight | < 18.5 | Low (but risks of other clinical problems increased) |
| Normal range | 18.5 - 24.9 | Average |
| Overweight | ≥ 25 | |
| Pre-obese | 25 - 29.9 | Increased |
| Obese class I | 30 - 34.9 | Moderate |
| Obese class I | 35 - 39.9 | Severe |
| Obese class I | ≥ 40 | Very severe |

Obesity has also been defined using the 85^{th} and 95^{th} BMI-percentiles as cutoffs for definition of obesity and severe obesity. BMI-percentiles have been calculated within several populations; centiles for the German population based on the German National Nutrition Survey have been available since 1994 (Hebebrand et al., 1994, 1996). Because the WHO classification of the different weight classes can only be applied to adults, it has become costumary to refer to BMI-percentiles for the definition of obesity in children and adolescents.

The recent rise in the prevalence of obesity is an issue of major concern for the health systems of several countries. In the USA the increase in the prevalence of all classes of obesity has been dated to the time period approximately between 1976 and 1990. During this time span, the prevalence of obesity increased by more than one half rising from 14.5% to 22.5%. Similar trends have been observed in other countries in Europe and South America.

Children and adolescents have not been exempt from this trend. Quite to the contrary, the increase in the USA has been substantial. Thus, between the 1960ies and 1990, overweight and obesity increased dramatically in 6 through to 17 year olds. The increments translate into relative increases of 40% using the 85^{th} BMI-centile (calculated in the 1960ies) as a cutoff and 100% upon use of the 95^{th} centile. In a cross sectional study of German children and adolescents treated as inpatients for extreme obesity between 1985 and 1995, we have reported a significant increase of the mean BMI of almost 2 kg/m² over this ten year period. Within this extreme group, the increments were most pronounced in the uppermost BMI ranges.

The mechanisms underlying this increase in the prevalence of obesity are unknown. Environmental factors have commonly been invoked as the underlying cause. Basically, both an increased caloric intake and a reduced level of physical activity have been discussed. In England the increase in obesity rates has been attributed to the latter mechanism. Thus, in this country, the average caloric intake even decreased somewhat within the last two decades, whereas indirect evidence stemming from the increases in hours spent watching television and from the average number of cars per household points to reduced levels of physical activity as the relevant causative factor.

Potentially life-threatening, chronic health problems associated with obesity fall into four main areas: 1) cardiovascular problems, including hypertension, chronic heart disease and stroke, 2) conditions associated with insulin resistance, namely Non-Insulin Dependent Diabetes Mellitus (NIDDM), 3) certain types of cancers, mainly the hormonally related and large-bowel cancers, and 4) gallbladder disease. Other problems associated with obesity include respiratory difficulties, chronic musculo-skeletal problems, skin problems and infertility (WHO, 1998).

The main currently available strategies for treating these disorders include dietary restriction, increments in physical activity, pharmacological and surgical approaches. In adults, long term weight loss is exceptional using conservative interventions. Present pharmacological interventions typically induce a weight loss of between five and fifteen kilograms; if the medication is discontinued, renewed weight gain ensues. Surgical treatments are comparatively successful and are reserved for patients with extreme obesity and/or with serious medical complications.

Recently, a 10 year old massively obese girl, in whom a leptin deficiency mutation had been detected, was treated successfully with recombinant leptin. This is the first individual who therapeutically profited from the detection of the mutation underlying her morbid obesity.

Several twin studies have been performed to estimate heritability of the BMI, some of which have encompassed over 1000 twin pairs. The results have been very consistent: The intrapair correlations among monozygotic twins were typically between 0.6 and 0.8, independent of age and gender. In one study, the correlations for monozygotic and dizygotic twins were basically the same, independent of whether the twins had been reared apart or together. Heritability of the BMI was estimated at 0.7; non-shared environmental factors explained the remaining 30% of the variance. Surprisingly, shared environmental factors did not explain a substantial proportion of the variance. Both hypercaloric and hypocaloric alimentation lead to similar degrees of weight gain or loss among both members of monozygotic twin pairs, indicating that genetic factors regulate the effect of environmentally induced variation of energy availability on body weight. Metabolic reactions and changes in body fat distribution upon overeating and undereating are also under genetic control (reviewed in Hebebrand et al., 1998).

A large adoption study has revealed that the BMI of adoptees is correlated with that of their biological parents and not with the BMI of the adoptive parents. Depending on the family study, the correlation between the BMI of sibs is between 0.2 and 0.4. Parent-offspring correlations are typically slightly lower. Segregation analyses have repeatedly suggested a major recessive gene effect. Based on these analyses, sample size calculations have been performed based on both concordant and discordant approaches. In contrast to the expectations, the concordant sib-pair approach was superior; a lower number of families were required to achieve the same power.

Family studies based on extremely obese young index patients, either mother or father or both, have a BMI > 90^{th} decile in the vast majority of the families. Based on index patients with a BMI > 95^{th} centile, approximately 20% of the respective families have a sib with a BMI > 90^{th} centile.

In conclusion, it is apparent that environmental factors interact with specific genotypes rendering an individual more or less susceptible to the development of obesity. Furthermore, despite the fact that major genes have been detected, it is necessary to consider that the spectrum reaches from such major genes to genes with an only minor influence.

The discovery of the leptin gene at the end of 1994 (Zhang et al., 1994) has been followed by a virtual explosion of scientific efforts to uncover the regulatory systems underlying appetite and weight regulation. It is currently the fastest growing biomedical field. This upswing has also resulted in large scaled molecular genetic activities which, due to obvious clinical interest, are basically all related to obesity in humans, rodents and other mammals (Hebebrand et al., 1998).

In this respect, many genes in which mutations lead to the presently known monogenic forms of obesity have been cloned in rodents. Systemic consequences of these mutations are currently being analysed. These models have provided insights into the complex regulatory systems involved in body weight regulation, the best known of which includes leptin and its receptor.

In mice, but also in pigs, over 15 quantitative trait loci (QTL) have been identified that are most likely relevant in weight regulation (Rankinen et al., 2002).

In humans, four exceedingly rare autosomal recessive forms of obesity have been described as of 1997. Mutations in the genes encoding for leptin, leptin receptor, prohormone convertase 1 and pro-opiomelanocortin (POMC) have been shown to cause massive obesity of an early onset type, associated with hyperphagia. Distinct additional clinical (e.g. red hair, primary amenorrhea) and/or endocrinological abnormalities (e.g. markedly altered serum leptin levels, lack of ACTH secretion) pinpointed to the respective candidate genes. Both the monogenic animal models and the human monogenic forms have led to new insights into the complex system underlying body weight regulation.

Very recently, the first autosomal dominant form of obesity was described in humans. Two different mutations within the melanocortin-4 receptor gene (*MC4R*) were observed to lead to extreme obesity in probands heterozygous for these variants. In contrast to the aforementioned findings, these mutations do not implicate readily obvious phenotypic abnormalities other than extreme obesity (Vaisse et al., 1998; Yeo et al., 1998). Interestingly, both groups detected the mutations by systematic screens in relatively small study groups (n=63 and n=43).

Hinney et al. (1999) screened the *MC4R* in a total of 492 obese children and adolescents. All in all, four individuals with two different mutations leading to haplo-insufficiency were detected. One was identical to that previously observed by Yeo et al. (1998). The other mutation, which was detected in three individuals, induced a stop mutation in the extracellular domain of the receptor. Approximately one percent of extremely obese individuals harbour haplo-insufficiency mutations in the *MC4R.* In addition to the two forms of haplo-insuffciency, Hinney et al. (1999) also detected additional mutations leading to both conservative and non-conservative amino acid exchanges. Interestingly, these mutations were mainly observed in the obese study group. The functional implications of these mutations are currently unknown.

The identification of individuals with *MC4R* mutations is interesting in the light of possible pharmacological interventions. Thus, intranasal application of adrenocorticotropin₄₋₁₀ (ACTH₄₋₁₀), representing a core sequence of all melanocortins, resulted in reduced weight, body fat mass and plasma leptin concentrations in healthy controls. The question arises as to how mutation carriers would react to this treatment, which could theoretically counterbalance their reduced receptor density.

The involvement of specific genes in weight regulation is further substantiated by data obtained from transgenic mice. For example, MC4R deficient mice develop early onset obesity (Huszar et al., 1997).

Different groups are conducting genome scans related to obesity or dependent phenotypes (BMI, leptin levels, fat mass, etc.). This approach appears very promising, because it is both systematic and model free. In addition, it has already been shown to be exceptionally successful. Thus, positive linkage results have been obtained even by analysing comparatively small study groups. More important, some findings have already been replicated. Each of the following regions has been identified by at least two independent groups: chromosome 1q32, chromosome 2p21, chromosome 10 and chromosome 20q13 (Rankinen et al., 2002).

WO95/17906 discloses a method of detecting predisposition to a disorder associated with any restriction polymorphism in the PPYR1 gene locus, and that there is available data that implicate this receptor in the control of obesity (pages 41 and 83).

WO96/14331 teaches that the third intracellular loop of PPYR1 receptor comprises a 27 aminoacid stretch spanning positions 236-263, and that this segment is essential for the interaction with the G-protein, a necessary step in the signal transduction cascade (pages 13-16). In addition, this document describes modified PPYR1 molecules in which some aminoacids are substituted in order disrupt the helix structure and block this interaction (page 30, line 3) and suggests the utility of said modified receptors in identifying compounds for the treatment of obesity (see Abstract and page 16).

### SUMMARY OF THE INVENTION

The present description now discloses the identification of a human obesity susceptibility gene, which can be used for the diagnosis, of obesity. The description more specifically demonstrates that certain alleles of the PPYR1 gene on chromosome 10 are related to susceptibility to obesity and represent novel targets for therapeutic intervention.

Herein described are several SNPs and mutations in the PPYR1 gene which are associated with obesity. Preferably, said SNPs associated with obesity are SNP718 and SNP826.

In particular, the present invention is directed to a method of detecting the presence of or predisposition to obesity in a subject, the method comprising (i) providing a sample from the subject and (ii) detecting the presence of an alteration in the PPYR1 gene locus in said sample, said alteration being SNP 718 (nucleotide 803 C/T in SEQ ID N°1).

Optionally, said alteration is detected through the genotyping of an haplotype, said haplotype comprising SNP 718 (nucleotide 803 C/T in SEQ ID N°1), SNP 826 (nucleotide 911 G/A in SEQ ID N°1) and a SNP selected from the group consisting of SNP 195 (nucleotide 280 G/A in SEQ ID N°1), SNP 588 (nucleotide 673 G/A in SEQ ID N°1), SNP 897 (nucleotide 982 C/T in SEQ ID N°1), SNP 948 (nucleotide 1033 C/T in SEQ ID N°1) and SNP 1047 (nucleotide 1132 C/T in SEQ ID N°1).

In one embodiment, the presence of an alteration in the PPYR1 gene locus is detected by sequencing, selective hybridisation and/or selective amplification.

In a further embodiment, said method comprises specifically amplifying PPYR1a gene, preferably with the amplification primer FN1A (SEQ ID No 9).

Optionally, the method comprises detecting the presence of an altered PPYR1 polypeptide, that comprises aminoacid polymorphism 240:Arg/Cys., preferably by contacting the sample with an antibody specific for said altered PPYR1 polypeptide and determining the formation of an immune complex.

The invention can be used in the diagnosis of predisposition to, obesity.

### LEGEND TO THE FIGURES

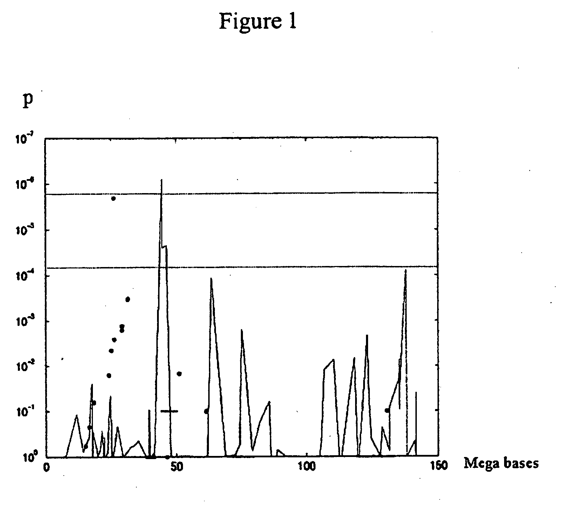

Figure 1 : Graphical presentation of the linkage peak on chromosome 10q11. The curves depict the linkage results for the GenomeHIP procedure, the dots depict results for microsatellite markers in the region. The dotted lines correspond to the Lander & Krygliak thresholds for suggestive evidence and evidence for linkage respectively. Linkage results obtained for 99 BAC clones on human chromosome 10 after the GenomeHIP^{R} procedure. Each point on the x-axis corresponds to a clone. Several clones are indicated by their library name for better orientation (e.g. RP11-70B16). The two horizontal lines at 3*10⁻⁴ and 2*10⁻⁵ for the p-values correspond to the significance levels for significant and suggestive linkage proposed by Krygliak and Lander for whole genome screens. The two highest peaks delimit the interval of clones with evidence for linkage after the GenomeHIP^{R} analysis.

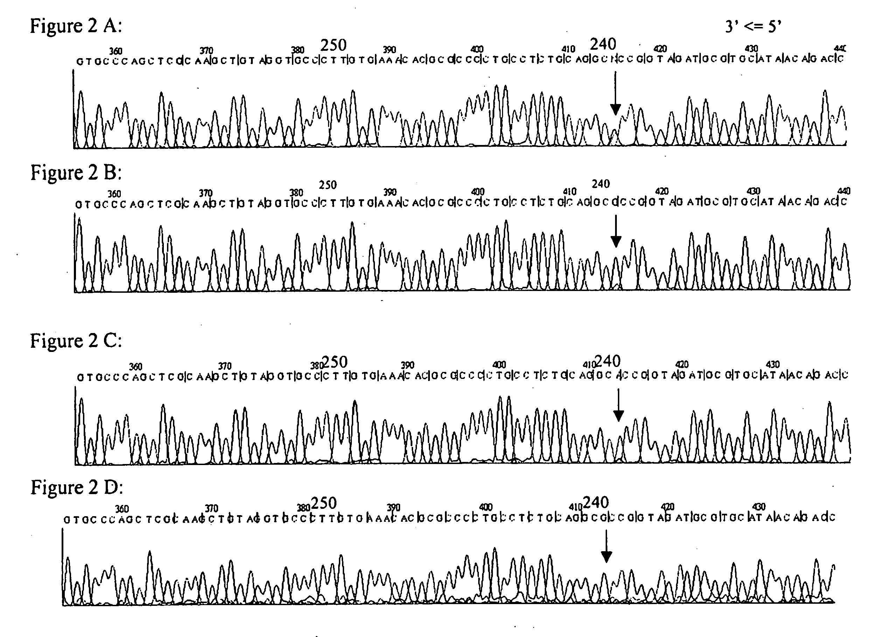

Figure 2 : Sequence example showing mutation at nucleotide position 718 of the human PPY1 recptor coding sequence. Figure 2A : example for a heterozygous mutation after pre-amplification with specific primer FN1A. The site of the mutation is indicated by the arrow. The codon triplets are deliminated by the vertical bars. Figure 2B : example for a heterozygous mutation after amplification with generic primers F5/R5. As in A the site of the mutation is indicated by the arrow. The codon triplets are deliminated by the vertical bars. As four alleles are present the mutant A allele is barely visible under the dominant G peak (small A peak under the G peak with app. 3:1 ratio). Figure 2C : Example for a homozygous mutation after pre-amplification with specific primer FN1A. Figure 2D : Homozygous wild type sequence for PPY1R after pre-amplification with specific primer FN1A.

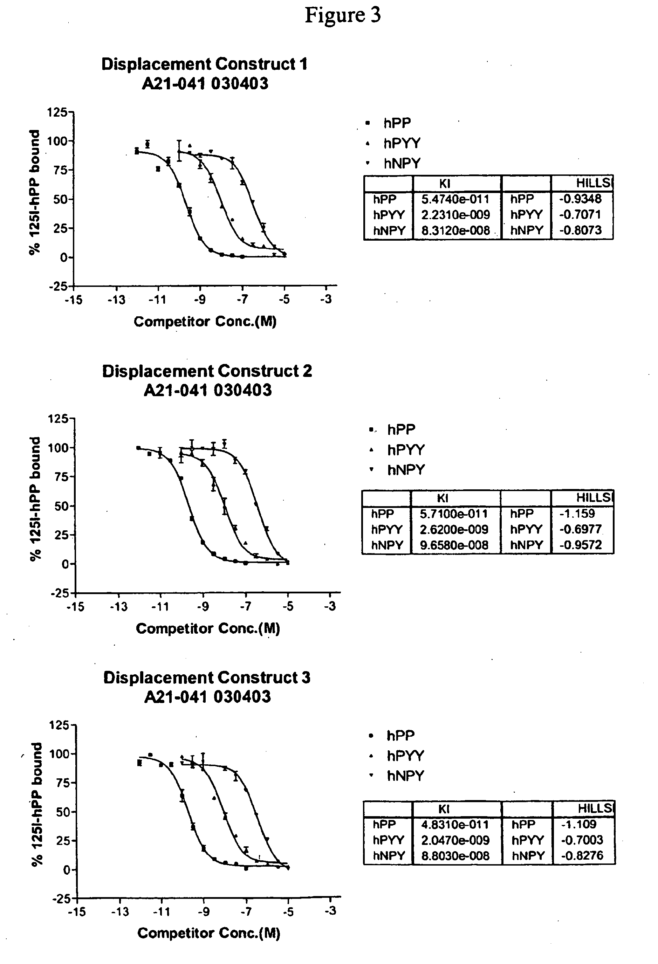

Figure 3 : Mutant receptor binding curves. Mutant receptor binding curves. The curves show results of competition replacement experiments using human PYY and NPY as competitors for pancreatic polypeptide (PP). Contructs 1, 2 and 3 correspond to the human PPY1 receptor wild-type, mutant 718 (codon 240, Arg-> Cys) and mutant 826 (codon 276, Val -> Met) respectively.

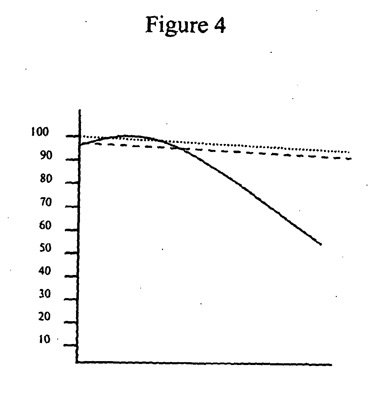

Figure 4 : second messanger (camp) response of the wild type and mutant receptors bearing mutations at positions 718 or 826 respectively. Results of cAMP measurements comparing the NPY4r wild-type receptor (solid curve) to the two mutant receptors bearing snp718 (dotted curve, 3^{rd} intracellular loop) and snp826 (broken curve, 6^{th} transmembrane domaine). Y-axis relative decrease of cAMP concentration arbitrary units.

### DETAILED DESCRIPTION OF THE INVENTION

The present invention discloses the identification of PPYR1 as a human obesity susceptibility gene. Various nucleic acid samples from 89 families with obesity were submitted to a particular GenomeHIP process. This process led to the identification of particular identical-by-descent fragments in said populations that are altered in obese subjects. By screening of the IBD fragments, we identified the pancreatic polypeptide Y receptor (PPYR1) gene as a candidate for obesity and related phenotypes. This gene is indeed present in the critical interval and expresses a functional phenotype consistent with a genetic regulation of obesity. Several mutated alleles and SNPs of the PPYR1 gene were also identified, as being correlated to obesity in human subjects. A particular point mutation in codon 240 (SNP 718 C/T) has been found in up to 60% of the tested obese subjects and is strongly associated with obesity (table 3). Other mutations were found, for instance, in codons 23, 40, 54, 99, 239, 247, 276, 287 and 343.

The present invention thus proposes to use PPYR1 gene and corresponding expression products for the diagnosis, of obesity.

### DEFINITIONS

Obesity and metabolic disorders: Obesity shall be construed as any condition of abnormal or excessive fat accumulation in adipose tissue, to the extent that health may be impaired. Associated disorders, diseases or pathologies include, more specifically, any metabolic disorders, including hypo-alphalipoproteinemia, familial combined hyperlipidemia, insulin resistant syndrome X or multiple metabolic disorder, coronary artery disease, diabetes mellitus and dyslipidemic hypertension. The invention may be used in various subjects, particularly human, including adults, children and at the prenatal stage.

Within the context of this invention, the PPYR1 gene locus designates all PPYR1 sequences or products in a cell or organism, including PPYR1 coding sequences, PPYR1 non-coding sequences (e.g., introns), PPYR1 regulatory sequences controlling transcription and/or translation (e.g., promoter, enhancer, terminator, etc.), as well as all corresponding expression products, such as PPYR1 RNAs (e.g., mRNAs) and PPYR1 polypeptides (e.g., a pre-protein and a mature protein).

As used in the present application, the term "PPYR1 gene" designates the human Pancreatic Polypeptide Receptor 1 gene on human chromosome 10, as well as variants, analogs and fragments thereof, including alleles thereof (e.g., germline mutations) which are related to susceptibility to obesity and metabolic disorders. The PPYR1 gene may also be referred to as the Y4 gene, the NPY4R gene or the PP 1 gene.

The term "gene" shall be construed to include any type of coding nucleic acid, including genomic DNA (gDNA), complementary DNA (cDNA), synthetic or semi-synthetic DNA, as well as any form of corresponding RNA. The term gene particularly includes recombinant nucleic acids encoding PPYR1, i.e., any non naturally occurring nucleic acid molecule created artificially, e.g., by assembling, cutting, ligating or amplifying sequences. A PPYR1 gene is typically double-stranded, although other forms may be contemplated, such as single-stranded. PPYR1 genes may be obtained from various sources and according to various techniques known in the art, such as by screening DNA libraries or by amplification from various natural sources. Recombinant nucleic acids may be prepared by conventional techniques, including chemical synthesis, genetic engineering, enzymatic techniques, or a combination thereof. Suitable PPYR1 gene sequences may be found on gene banks, such as Unigene Cluster for PPYR1 (Hs.54426), Unigene Representative Sequence NM_005972, REFSEQ mRNAs: NM-005972, MIPS assembly: H8600S1, DOTS assembly: DT.406393 and additional gene/cDNA sequences: U35232, U42387, Z66526.

A particular example of a PPYR1 gene comprises SEQ ID No: 1.

The term "PPYR1 gene" includes any variant, fragment or analog of SEQ ID No:1 or of any coding sequence as identified above. Such variants include, for instance, naturally-occurring variants due to allelic variations between individuals (e.g., polymorphisms), mutated alleles related to obesity, alternative splicing forms, etc. The term variant also includes PPYR1 gene sequences from other sources or organisms. Variants are preferably substantially homologous to SEQ ID No:1, i.e., exhibit a nucleotide sequence identity of at least about 65%, typically at least about 75%, preferably at least about 85%, more preferably at least about 95% with SEQ ID No:1. Variants and analogs of a PPYR1 gene also include nucleic acid sequences, which hybridize to a sequence as defined above (or a complementary strand thereof) under stringent hybridization conditions. Typical stringent hybridisation conditions include temperatures above 30° C, preferably above 35°C, more preferably in excess of 42°C, and/or salinity of less than about 500 mM, preferably less than 200 mM. Hybridization conditions may be adjusted by the skilled person by modifying the temperature, salinity and/or the concentration of other reagents such as SDS, SSC, etc.

A fragment of a PPYR1 gene designates any portion of at least about 8 consecutive nucleotides of a sequence as disclosed above, preferably at least about 15, more preferably at least about 20 nucleotides, further preferably of at least 30 nucleotides. Fragments include all possible nucleotide length between 8 and 100 nucleotides, preferably between 15 and 100, more preferably between 20 and 100.

A PPYR1 polypeptide designates any protein or polypeptide encoded by a PPYR1 gene as disclosed above. The term "polypeptide" refers to any molecule comprising a stretch of amino acids. This term includes molecules of various length, such as peptides and proteins. The polypeptide may be modified, such as by glycosylations and/or acetylations and/or chemical reaction or coupling, and may contain one or several non-natural or synthetic amino acids. A specific example of a PPYR1 polypeptide comprises all or part of SEQ ID No:2 or a variant thereof.

### DIAGNOSIS

Within the context of the present invention, the term 'diagnosis" includes the detection, monitoring, dosing, comparison, etc., at various stages, including early, pre-symptomatic stages, and late stages, in adults, children and pre-birth. Diagnosis typically includes the prognosis, the assessment of a predisposition or risk of development, the characterization of a subject to define most appropriate treatment (pharmaco-genetics), etc.

A particular object of this invention resides in a method of detecting the presence of or predisposition to obesity in a subject, the method comprising detecting in a sample from the subject the presence of an alteration in the PPYR1 gene locus said alteration being SWP718 (nucleotide 803 C/T in SEQ ID NO:1) the forward primer FN1A (SEQ ID No 9) allows a specific amplification of PPYR1a gene (see Examples).

The alteration may be determined at the level of the PPYR1 gDNA, RNA or polypeptide. Optionally, the detection is performed by sequencing all or part of the PPYR1 gene or by selective hybridisation or amplification of all or part of the PPYR1 gene. More preferably a PPY1R gene specific amplification is carried out before the alteration identification step.

In this regard, the present invention now discloses several SNPs and mutations in the PPYR1 gene, which are associated with obesity. These point mutations are reported in the following table 2.

**Table 2**

| | Numbering in SEQ ID No: 1 | Polymorphism | Codon and/or Amino acid change | Freq % |
|---|---|---|---|---|
| SNP 67 | 152 | C/T | Pro 23 -> Ser | 0.30 |
| SNP 118 | 203 | G/A | Val 40 -> Met | 0.30 |
| SNP 160 | 245 | G/A | Val 54 -> Met | 0.30 |
| SNP 195 | 280 | G/A | 65 | |
| SNP 295 | 380 | G/T | Ala99 -> Ser | |
| SNP 588 | 673 | G/A | 196 | |
| SNP 691 | 776 | C/T | 231 | |
| SNP 715 | 800 | C/T | Arg239 -> Trp | 1 |
| SNP 716 | 801 | G/A | Arg239 -> Glu | 1.5 |
| SNP 718 | 803 | C/T | Arg240 -> Cys | 36 |
| SNP 739 | 824 | G/A | Val 247 -> Met | 0.30 |
| SNP 826 | 911 | G/A | Val276 ->Met | 15 |
| SNP 860 | 945 | T/C | Leu 287 -> Pro | 0.30 |
| SNP 897 | 982 | C/T | 299 | |
| SNP 948 | 1033 | C/T | 316 | |
| SNP 1029 | 1114 | C/A | Ser 343 -> Arg | 0.30 |
| SNP 1047 | 1132 | G/A | 349 | |

These point mutations affect the coding region of the PPYR1 gene and result, for some of them, in an altered amino acid residue in the encoded polypeptide. These point mutations have been detected in subjects having obesity. Haplotypes were constructed for all these SNPs to determine the naturally occurring phase for each possible SNP combination. The results show that SNP 588 is in almost complete linkage disequilibrium with all other occurring non-coding SNPs and can be used as a reliable denominator for the different haplotypes involving the amino acid changes. Haplotypes comprising SNP 588 and any combination of amino acid changing SNPs were then used to test for association between all the resulting alleles and obesity. The results show that haplotypes characterized by a point mutation in codon 240 (see table 2 above) are strongly associated with obesity (tables 3 and 4).

As indicated above, various techniques known in the art may be used to detect or quantify altered PPYR1 gene or sequence, including sequencing, hybridisation, amplification and/or binding to specific ligands (such as antibodies). Other suitable methods include allele-specific oligonucleotide (ASO), allele-specific amplification, Southern blot (for DNAs), Northern blot (for RNAs), single-stranded conformation analysis (SSCA), PFGE, fluorescent in situ hybridization (FISH), gel migration, damped denaturing gel electrophoresis, heteroduplex analysis, RNase protection, chemical mismatch cleavage, ELISA, radio-immunoassays (RIA) and immuno-enzymatic assays (IEMA).

Some of these approaches (e.g., SSCA and CGGE) are based on a change in electrophoretic mobility of the nucleic acids, as a result of the presence of an altered sequence. According to these techniques, the altered sequence is visualized by a shift in mobility on gels. The fragments may then be sequenced to confirm the alteration.

Some others are based on specific hybridisation between nucleic acids from the subject and a probe specific for wild-type or altered PPYR1 gene or RNA. The probe may be in suspension or immobilized on a substrate. The probe is typically labelled to facilitate detection of hybrids.

Some of these approaches are particularly suited for assessing a polypeptide sequence such as Northern blot, ELISA and RIA. These latter require the use of a ligand specific for the polypeptide, more preferably of a specific antibody.

### Sequencing:

Sequencing can be carried out using techniques well known in the art, using automatic sequencers. The sequencing may be performed on the complete PPYR1 gene or, more preferably, on specific domains thereof, typically those known or suspected to carry deleterious mutations or other alterations.

### Amplification

Amplification is based on the formation of specific hybrids between complementary nucleic acid sequences that serve to initiate nucleic acid reproduction.

Amplification may be performed according to various techniques known in the art, such as by polymerase chain reaction (PCR), ligase chain reaction (LCR), strand displacement amplification (SDA) and nucleic acid sequence based amplification (NASBA). These techniques can be performed using commercially available reagents and protocols. Preferred techniques use allele-specific PCR or PCR-SSCP. Amplification usually requires the use of specific nucleic acid primers, to initiate the reaction.

Nucleic acid primers useful for amplifying sequences from the PPYR1 gene or locus are able to specifically hybridize with a portion of the PPYR1 gene locus that flank a target region of said locus, said target region being altered in certain subjects having obesity or associated disorders and said target regions being provided in Table 2 above. More particularly, the amplification is specific of the PPYR1 a gene.

Specific examples of primers that can be used to amplify the PPYR1 target region comprising SNP718 have a sequence identical or complementary to a portion of SEQ ID No: 1 located between nucleotide residues 700 to 800 or 805 to 900.

Primers that can be used to amplify PPYR1 target region comprising other SNPs as identified in table 2 may be designed based on the sequence of SEQ ID No: 1.

Primers that can be used to specifically amplify the PPYR1 target region comprising SNP718 should, for instance, comprise a sequence identical or complementary to a portion of SEQ ID No:1 located between nucleotide residues 800 and 805. A specific example of such a primer comprises the sequence CGGTGC (nucleotide residues 800-805 of SEQ ID No:1 with SNP718).

The description concerns a nucleic acid primer, wherein said primer is complementary to and hybridizes specifically to a portion of a PPYR1 gene or RNA, wherein said portion comprises, a nucleotide T at position 801 and/or a nucleotide A at position 911 of SEQ ID No 1.

Typical primers are single-stranded nucleic acid molecules of about 5 to 60 nucleotides in length, more preferably of about 8 to about 25 nucleotides in length. The sequence can be derived directly from the sequence of the PPYR gene locus. Perfect complementarity is preferred, to ensure high specificity. However, certain mismatch may be tolerated.

### Selective hybridization

Hybridization detection methods are based on the formation of specific hybrids between complementary nucleic acid sequences that serve to detect nucleic acid sequence alteration(s).

A particular detection technique involves the use of a nucleic acid probe specific for wild-type or altered PPYR1 gene or RNA, followed by the detection of the presence of a hybrid. The probe may be in suspension or immobilized on a substrate or support (as in nucleic acid array or chips technologies). The probe is typically labelled to facilitate detection of hybrids.

In this regard, a particular embodiment of this invention comprises contacting the sample from the subject with a nucleic acid probe specific for an altered PPYR1 gene locus, and assessing the formation of an hybrid. In a particular, preferred embodiment, the method comprises contacting simultaneously the sample with a set of probes that are specific, respectively, for wild type PPYR1 gene locus and for various altered forms thereof. In this embodiment, it is possible to detect directly the presence of various forms of alterations in the PPYR1 gene locus in the sample. Also, various samples from various subjects may be treated in parallel.

Within the context of this invention, a probe refers to a polynucleotide sequence which is complementary to and capable of specific hybridisation with a (target portion of a) PPYR1 gene or RNA, and which is suitable for detecting polynucleotide polymorphisms associated with PPYR1 alleles which predispose to or are associated with obesity or metabolic disorders. Probes are preferably perfectly complementary to the PPYR1 gene, RNA, or target portion thereof. Probes typically comprise single-stranded nucleic acids of between 8 to 1000 nucleotides in length, for instance of between 10 and 800, more preferably of between 15 and 700, typically of between 20 and 500. It should be understood that longer probes may be used as well. A preferred probe is a single stranded nucleic acid molecule of between 8 to 500 nucleotides in length, which can specifically hybridise to a region of a PPYR1 gene or RNA that carries an alteration.

A specific probe is a nucleic acid probe specific for an altered (e.g., a mutated) PPYR1 gene or RNA, i.e., a nucleic acid probe that specifically hybridises to said altered PPYR1 gene or RNA and essentially does not hybridise to a PPYR1 gene or RNA lacking said alteration. Specificity indicates that hybridisation to the target sequence generates a specific signal which can be distinguished from the signal generated through non-specific hybridisation. Perfectly complementary sequences are preferred to design probes. It should be understood, however, that certain mismatch may be tolerated, as long as the specific signal may be distinguished from non-specific hybridisation.

Particular examples of such probes are nucleic acid sequences complementary to a target portion of the PPYR1 gene or RNA carrying a point mutation as listed in Table 2 above, more preferably a nucleotide T at position 152 (SNP 67), a nucleotide A at position 203 (SNP118), a nucleotide A at position 245 (SNP160), a nucleotide T at position 800 (SNP715), a nucleotide A at position 801 (SNP716), a nucleotide A at position 824 (SNP739), a nucleotide A at position 911 (SNP826), a nucleotide C at position 945 (SNP860), and/or a nucleotide A at position 1114 (SNP1029) of SEQ ID No 1 or a combination thereof, still more preferably a nucleotide T at position 800, a nucleotide A at position 801 and/or a nucleotide A at position 911 of SEQ ID No 1 or a combination thereof.

The sequence of the probes can be derived from the sequences of the PPYR1 gene and RNA as provided in the present application. Nucleotide substitutions may be performed, as well as chemical modifications of the probe. Such chemical modifications may be accomplished to increase the stability of hybrids (e.g., intercalating groups) or to label the probe. Typical examples of labels include, without limitation, radioactivity, fluorescence, luminescence, enzymatic labelling, etc.

### Specific Ligand Binding

As indicated above, alteration in the PPYR1 gene locus may also be detected by screening for alteration(s) in PPYR1 polypeptide that comprises amino acid polymorphism 240: Arg/Cys. In this regard, a specific embodiment of this invention comprises contacting the sample with a ligand specific for a PPYR1 polypeptide that comprises amino acid polymorphism 240: Arg/Cys and determining the formation of a complex.

Different types of ligands may be used, such as specific antibodies. In a specific that comprises amino acid polymorphism 240: Arg/Cys embodiment, the sample is contacted with an antibody specific for a PPYR1 polypeptide and the formation of an immune complex is determined. Various methods for detecting an immune complex can be used, such as ELISA, radio-immunoassays (RIA) and immuno-enzymatic assays (IEMA).

Within the context of this invention, an antibody designates a polyclonal antibody, a monoclonal antibody, as well as fragments or derivatives thereof having substantially the same antigen specificity. Fragments include Fab, Fab'2, CDR regions, etc. Derivatives include single-chain antibodies, humanized antibodies, poly-functional antibodies, etc.

An antibody specific for a PPYR1 polypeptide that comprises amino acid polymorphism 240: Arg/Cys designates an antibody that selectively binds a PPYR1 polypeptide that comprises amino acid polymorphism 240: Arg/Cys i.e., an antibody raised against a PPYR1 polypeptide that comprises amino acid polymorphism 240: Arg/Cys or an epitope-containing fragment thereof. Although non-specific binding towards other antigens may occur, binding to the target PPYR1 polypeptide that comprises amino acid polymorphism 240: Arg/Cys occurs with a higher affinity and can be reliably discriminated from non-specific binding.

In a specific embodiment, the method comprises contacting a sample from the subject with (a support coated with) an antibody specific for an altered form of a PPYR1 polypeptide, that comprises amino acid polymorphism 240: Arg/Cys and determining the presence of an immune complex. In a particular embodiment, the sample may be contacted simultaneously, or in parallel, or sequentially, with various (supports coated with) antibodies specific for different forms of a PPYR1 polypeptide such as a wild-type and various altered forms thereof.

Particular examples of such specific ligands are antibodies specific for altered PPYR1 polypeptide sequence resulting from mutations listed in Table 2 above. More particularly these ligands are specific of any mutations in codons 23, 40, 54, 239, 240, 247, 276, 287 and 343as listed in table 2 or any combination of those mutations. More preferably, said ligands are specific of any mutations in codons 239, 240 and/or 276 listed in Table 2 above.

The diagnosis methods can be performed in vitro or ex vivo. They use a sample from the subject, to assess the status of the PPYR1 gene locus. The sample may be any biological sample derived from a subject, which contains nucleic acids or polypeptides. Examples of such samples include fluids, tissues, cell samples, organs, biopsies, etc. Most preferred samples are blood, plasma, saliva, urine, seminal fluid, etc. Prenatal diagnosis may also be performed by testing foetal cells or placental cells, for instance The sample may be collected according to conventional techniques and used directly for diagnosis or stored. The sample may be treated prior to performing the method, in order to render or improve availability of nucleic acids or polypeptides for testing. Treatments include, for instant, lysis (e.g., mechanical, physical, chemical, etc.), centrifugation, etc. Also, the nucleic acids and/or polypeptides may be pre-purified or enriched by conventional techniques, and/or reduced in complexity. Nucleic acids and polypeptides may also be treated with enzymes or other chemical or physical treatments to produce fragments thereof. Considering the high sensitivity of the claimed methods, very few amounts of sample are sufficient to perform the assay.

As indicated, the sample is preferably contacted with reagents such as probes, primers or ligands in order to assess the presence of an altered PPYR1 gene locus. Contacting may be performed in any suitable device, such as a plate, tube, well, glass, etc. In specific embodiments, the contacting is performed on a substrate coated with the reagent, such as a nucleic acid array or a specific ligand array. The substrate may be a solid or semi-solid substrate such as any support comprising glass, plastic, nylon, paper, metal, polymers and the like. The substrate may be of various forms and sizes, such as a slide, a membrane, a bead, a column, a gel, etc. The contacting may be made under any condition suitable for a complex to be formed between the reagent and the nucleic acids or polypeptides of the sample.

The finding of an altered PPYR1 polypeptide, RNA or DNA in the sample as claimed is indicative of the presence of an altered PPYR1 gene locus in the subject, which can be correlated to the predisposition of obesity. For example, an individual having a germline PPYR1 mutation as claimed has an increased risk of developing obesity or metabolic disorders.

Further aspects and advantages of the present invention will be disclosed in the following experimental section, which should be regarded as illustrative and not limiting the scope of the present application.

### EXAMPLES

### 1. Identification of an Obesity susceptibility locus on human chromosome 10

### A. Linkage studies

Hager et al. (1998) first identified a locus on human chromosome 10 linked to massive human obesity. The linkage study showed evidence for linkage between markers D10S191 and D10S220 with a MLS maximum at marker D10S197. The whole region covered an interval of approximately 39 centimorgan.
The locus on chromosome 10p may account for 21-36 % of the obesity in the study group analysed by the French group (Hager et al., 1998).
Hinney at al. (2000) replicated the linkage to chromosome 10. For this purpose, 94 German families with two obese children were genotyped for 12 microsatellite markers in the region previously identified by Hager et al. (1998). The MLS is 2.3. A further study (Price RA, 2001) independently confirmed linkage of this locus in an American population. It is therefore currently safe to conclude that a substantial number of obese families harbour (an) allele(s) predisposing to obesity in a gene on chromosome 10.

### B. GenomeHIP platform to identify the chromosome 10 susceptibility gene

As outlined above, the chromosomal interval of the initial linkage findings is huge (39 cM), not allowing a positional cloning approach to identify the obesity susceptibility gene in this region. We applied our GenomeHIP platform to reduce the region to a small interval that would allow rapid identification of the obesity susceptibility gene.
Briefly, the technology consists of forming pairs from the DNA of related individuals. Each DNA is marked with a specific label allowing its identification. Hybrids are then formed between the two DNAs. A particular process (WO00/53802) is then applied that selects all fragments identical-by-descent (IBD) from the two DNAs in a multi step procedure. The remaining IBD enriched DNA is then scored against a BAC clone derived DNA microarray that allows the positioning of the IBD fraction on a chromosome.
The application of this process over many different families results in a matrix of IBD fractions for each pair from each family. Statistical analyses then calculate the minimal IBD regions that are shared between all families tested. Significant results (p-values) are evidence for linkage of the positive region with the trait of interest (here obesity). The linked interval can be delimited by the two most distant clones showing significant p-values.
In the present study, 89 families of German origin (117 independent sib-pairs) concordant for massive obesity (as defined by a body mass index > 90%ile) were submitted to the GenomeHIP process. The resulting IBD enriched DNA fractions were then labelled with Cy5 fluorescent dyes and hybridised against a DNA array consisting of 99 chromosome 10 derived human BAC clones resulting in an average chromosome-wide resolution of 250 kilobases. Non-selected DNA labelled with Cy3 was used to normalise the signal values and compute ratios for each clone. Clustering of the ratio results were then performed to determine the IBD status for each clone and pair.
By applying this procedure, several BAC clones spanning an approximately 4 mega-base region on chromosome 10 (bases 44000000 to 48000000) were identified, that showed significant evidence for linkage to obesity (table 2).

**Table 3: Linkage results for chromosome 10q11 in the region of PPY1R: Indicated is the region correspondent to 4 BAC clones with evidence for linkage. The start and stop positions of the clones correspond to their genomic location respective to the start of the chromosome (p-ter).**

| Chr. | Clone | cMorgan | % genotypes | p- value | Start | Stop |
|---|---|---|---|---|---|---|
| 10 | RP11-358L16-UA221 | 14.03 | 96.55 | 0.987 | | |
| 10 | RP11-534N5-UA221 | 14.1 | 93.1 | 0.632 | | |
| 10 | RP11-175I17-UA221 | 14.1 | 91.38 | 0.114 | | |
| 10 | RP11-556L1-UA221 | 14.21 | 74.14 | 0.000491 | 46466063 | 46637992 |
| 10 | RP11-314P12-UA221 | 14.5 | 89.66 | 0.0014 | 46942672 | 47017702 |
| 10 | RP11-508N16-UA22 | 115.73 | 82.76 | 0.0000010* | 48319411 | 48319498 |
| 10 | RP11-70E21-UA221 | 16.39 | 67.24 | 0.000013* | 49276409 | 49449647 |

### C. Identification of an obesity susceptibility gene on chromosome 10

By screening the aforementioned 4 mega-base chromosomal region, we identified the pancreatic polypeptide Y receptor (PPYR1) gene as a candidate for obesity and related phenotypes. This gene is indeed present in the critical interval, with evidence for linkage delimited by the clones outlined in B. above.

PPYR1 is an intronless gene that encodes a predicted 375-amino acid polypeptide having highest homology to the Y1 receptor gene (42% amino acid identity). PPY1R is a member of the neuropeptide Y receptor family that so far comprises 5 members identified in humans (NPY1R, NPY2R, NPY4R (PPY1R), NPY5R and NPY6R) that show specific affinities to a family of neuropeptides (NPY, PPY and PP). Almost all these peptides and receptors have been shown to play a role in regulating energy metabolism (food intake and energy expenditure regulation). Of all the receptors PPY1R is the only one that is primarily expressed in peripheral tissues (gut, adipose tissue) while the others are primarily expressed in different parts of the brain. It has been shown in several studies that impairment of the PPY1 receptor is involved in malfunction of food intake regulation and weight gain in animal studies. The physiological role of the peripheral PPY1 receptor seems to lie in an inhibitory signal following binding of its physiological ligand pancreatic polypeptide (PP) thereby activating a cascade that inhibits food-intake in response to this satiety factor. Mutations in the receptor could therefore decrease the impact of the satiety factor PP and be involved in the aetiology of obesity.

Taken together, the linkage results provided in the present application, identifying the human PPYR1 gene in the critical interval of genetic alterations linked to obesity on chromosome 10, with its putative involvement in the NPY/PP signalling pathways, we conclude that alterations (e.g., mutations and/or polymorphisms) in the PPYR1 gene or its regulatory sequences may contribute to the development of human obesity and represent a novel target for diagnosis or therapeutic intervention.

### 2. SNP identification

90 families with at least one individual wit extreme, early onset obesity and their parents were included in the mutation screen. In addition, 10 multigenerational pedigrees from the CEPH pool of families were screened for mutations in the PPYR1 gene to allow the unambiguous determination of multi-SNP haplotypes.

Primers were designed to amplify the single exon of the PPYR1 gene in three overlapping fragments. The sequence of the primers is provided below:

| primer | sequence | annealing | fragment | SEQ ID |
|---|---|---|---|---|
| Reverse 5 | TAAGTGGAAGAGCCTGGCA | 60 °C | | 3 |
| Forward 5 | CCTGGAGAATGTCTTCCACAA | 60 °C | size:642 pb | 4 |
| | | | | |
| Reverse 6 | AGGTGGTGTAGATGGTGCG | 60 °C | | 5 |
| Forward 6 | TTCTCTGAACATTGCCAGGAT | 60 °C | size:561 pb | 6 |
| | | | | |
| Reverse 7 | AGTCCATGATGGTGTAGACGG | 60 °C | | 7 |
| Forward 7 | AGCATGCAACAAGTGCTTCTAA | 60 °C | size:568 pb | 8 |

The resulting amplification products were directly sequenced by dye-terminator sequencing to identify mutations and polymorphisms in the gene. Ambiguous samples were re-amplified and re-sequenced by dye-terminator and dye-primer sequencing.

A total of 12 single nucleotide polymorphisms were detected in the gene (for positions see table 2). Four of these resulted in changes of the amino-acids in the respective codons, as illustrated in table 2.

Haplotypes for all 12 SNPs were constructed to identify the phase for all SNPs. Several SNPs (see haplotye listing) were in complete linkage disequilibrium (LD). For these, one SNP (SNP 588) representative for the LD group was selected for further analyses of the haplotypes. Finally three SNPs were selected for haplotype association analysis. Haplotypes were analysed in two ways:
for linkage as a multi-allelic marker and
for association using all haplotypes identified to carry out a transmission disequilibrium test (TDT).

The TDT is a powerful association test as it is insensitive to population stratification problems in the tested sample. Briefly, the segregation of alleles from heterozygous parents to their affected off-spring is tested. The portion of alleles transmitted to the affected offspring compared to the non-transmitted alleles is compared to the ratio expected under random distribution. A significant excess of allele transmission over the expected value is evidence for an association of the respective allele with the phenotype (here obesity) in question.

Briefly 400 trio families were genotyped for three SNPs (588, 718 and 826). Haplotypes were constructed using the GENEHUNTER package for TDT (version 2.1_r2). The results of this analysis show that certain alleles or haplotypes, all characterized by the presence of a mutation at position 718 in the coding region (codon 240 that results in an amino acid change from Arg to Cys) are strongly associated with obesity. In the tested population, haplotypes having SNP718 are strongly correlated to obesity (132 transmitted vs 90 non-transmitted, Chi2 = 7.6, p = 0.004, as determined by TDT), while haplotypes devoid of SNP718 are not transmitted preferentially to obese subjects (Chi2 = 2.48, p= 0.12). Indeed, haplotypes that do not carry SNP718 show a tendency to be under-represented in obese subjects (95 transmitted vs 118 non-transmitted), strengthening the statistical evidence for this mutation to be involved in the development of obesity in these subjects. Examples of haplotypes with preferential transmission of SNP718 to obese individuals are given in table 3.

The SNPs in codon 239 leading to changes of the amino acid as described in table 2are rarer than SNP718 but also show a statistical tendency to be preferentially transmitted to obese subjects.

**Table 4**

| Haplotype # | Haplotype code | SNP Sequence | transmitted | Non-trans mitted |
|---|---|---|---|---|
| HAPLO_1 | 1 1 1 | ACG | 95 | 118 |
| HAPLO_2 | 1 1 2 | ACA | 4 5 | |
| HAPLO_3 | 2 1 1 | G C G | 75 | 82 |
| HAPLO_4 | 2 1 2 | G C A | 57 | 54 |
| | | | | |
| HAPLO_5 | 221 | GTG | 132 | 90 |
| HAPLO_6 | 222 | G T A | 7 | 7 |
| | | | | |

Legend: haplotypes are listed in 5' - 3' direction. 1 and 2 denote the two nucleotide possibilities for each SNP as listed in table 2. The positions 1-3 of the SNPs listed correspond to SNPs 588, 718 and 826 respectively as listed in table 2. Other SNPs were not included because they were in complete linkage disequilibrium with at least one of the seven SNPs in the haplotype. SNPs representing rare mutations were not tested individually as their low frequency does not permit meaningful association studies a list of these mutations is shown in table 3 with their respective frequencies in the study group.

### PCR conditions for locus specific PCR and nested PCR

Locus specific PCR was performed for each individual to specifically amplify only the PPYR1a or PPYR1bis locus. The PPYR1a locus was amplified using the reverse primer FN1A (5'-CAGGGCTCTAGCACATGAAT-3' SEQ ID No 9) in combination with the forward primer 7 (SEQ ID No 8) and the PPYR1bis locus was amplified using the reverse primer FN1G (5'-CAGGGCTCTAGCACATGAAC-3' SEQ ID No 10) in combination with the same forward primer 7. Amplification reactions were performed in 96 well plates in a 25 µl reaction volume on a DYAD (MJ Research) using the Expand Long Template PCR System (Roche Molecular Biochemicals), 7.5 pmol each forward and reverse primer, 175 µM dNTPs, 1.25 units Expand enzyme mix (Roche Molecular Biochemicals) and 25 ng genomic DNA. After 3 min at 94°C, amplification was carried out for 35 cycles comprising of 30 sec at 94°C, 30 sec at 58°C, 4 min at 68°C, followed by incubation at 72°C for 10 min.

The reactions of the locus specific PCR were diluted 1:100 in water in a 96 well plate. An aliquot of 2 µl of the diluted locus specific PCR reactions was used in nested PCR reactions with primers F5 (SEQ ID No 3) and R5 (SEQ ID No 4). PCR reactions were performed in 96 well plates in a 20 µl volume on a Perkin Elmer 9600 containing 1 x GeneAMP PCR buffer II (Applied Biosystems), 5 pmol each forward and reverse primer, 200 µM dNTPs, 2 mM MgCl₂, 1.25 units AmpliTaq Gold polymerase 'Applied Biosystems) and 2 µl 1:100 diluted locus specific PCR product. After heating at 94°C for 5 min, amplification was carried out for 30 cycles. Each cycle comprises: 30 sec at 94°C, 30 sec at 60°C, and 40 sec at 72°C, followed by a final incubation for 10 min at 72°C.

### Functional studies of PPYR1 receptor mutations 718 and 826

### 1) PCR

Amplification of NPY4(a/bis) receptor coding sequence from 3 different genomic DNAs by PCR (each individual being homozygous for either position 718 or 826 of the coding sequence of NPY4a receptor respectively). The goal was to obtain 3 different constructs:
One with the wild type sequence at positions 718 and 826 (718-C and 826-G)
One with the variation at position 718 (718-T, Arg-Cys)
One with the variation at the position 826 (826-A (Val-Met)
The forward primer contains (from 5' to 3'):
- a non-specific-tailing-sequence (6 bases)
- the sequence of the restriction site Xho I (CTGGAG)
- and 12 bases complementary to the start coding sequence including the ATG start codon (tccactATGaac)
The reverse primer contains (from 5' to 3'):
- a non-specific-tailing-sequence (6 bases)
- the sequence of the restriction site Pme I (GTTTAAAC)
- and 20 bases downstream of the TAA codon, starting 7 bases after this stop codon.
PCR was performed using Expand Taq Polymerase from Roche Diagnostics.

### 2) Digestion of PCR products

The obtained PCR products (around 1200 bp) were digested with Xho I and PmeI simultaneously overnight at 37°C and purified using the Qiaquick PCR purification kit.

### 3) Preparation of the vector (pcDNA3.1/myc-HIS version A, from Invitrogen N°V800-20).

This vector was digested with Xho I and PmeI simultaneously overnight at 37°C. The digested vector was loaded on an agarose gel, and the band corresponding to the digested vector (5400 bp) was excised from the gel. The vector was then purified by using the Qiaquick gel extraction kit.

### 4) Ligation

The digested PCR fragments and the digested vector were ligated using the T4 DNA Ligase (from Promega) overnight at 4°C.

### 5) Transformation

A competent E Coli strain (JM109) was transformed with the ligation mixtures using a heat shock method (30 min on ice, heat 30sec at 42°c and 1 min on ice). The selection of transformed bacteria was carried out on LB plates containing ampicillin.

### 6) Screening of clones

The clones were screened by colony-PCR using the primers T7 Forward and BGH Reverse. Only the clones that contained PCR products of the expected length (1.2 kb) were conserved.

### 7) Sequencing step

The conserved clones were sequenced using the same primers. For each construct, one clone containing the expected sequence was conserved.

### 8) Plasmid Purification

One positive clone for each construct was selected and a classical Midiprep (Qiagen kit) was carried out. The DNA concentration was established by UV spectro-photometry analysis, and 150µg of each construct was precipitated in 150 µL Ethanol (100%).

### Functional Evaluation

For the three samples (wt plus two mutant alleles) precipitate was collected (supernatant was saved) and dissolved in 150 ul water. (Expected conc 1ug/ul.)

One plate of COS cells was transfected for each construct with 12 ul (supposedly 12 ug) plasmid to obtain a transient cell line for each construct. Plasmid (12 ul) cleavage was started with SspI. Transfected cells were harvested and frozen at -80.

Test binding was performed with different membrane dilutions to confirm binding and to determine what dilution would be necessary. Radioligand: iodinated hPP.

Receptor membrane quantities were determined for competition experiments.

Transient transfections. Two plates for each plasmid based upon new determination of concentration. Larger volumes of plasmed solutions. Transfected quantities:
Construct 1: 1.4 ug
Construct 2: 1.4 ug
Construct 3: 1.8 ug

pUC control plasmid: 0.1ug/ul as expected, thus measurements were judged reliable.
Concentrations were verified on agarose gel.
Plasmids were transformed into competent bacteria, strain DH5-alpha, to be able to prepare plasmid DNA for additional transfections.

Plasmid maxi preps performed. Final volume 400 ul.
Construct 1: 1.4 ug/ul
Construct 2: 1.3 ug/ul
Construct 3: 1.3 ug/ul

### Competition experiments: One competition curve for each construct (duplicate samples). Radioligand hPP. Competing ligand hPYY.

Competition experiments on membranes from transfection 2: One curve for each construct (including hY4-pTEJ) and each of the ligands hPP, hPYY and hNPY.

### Evaluation of competition experiments (see table 5 and Fig 3).

Conclusion: No apparent difference between the two mutants and the wildtype. Construct 2 gave quite variable results for hPYY with very high SEM. The experiment has been repeated 6 times.

**Table 5 : Competition Statistics**

| | Ki | ±SEM | n |
|---|---|---|---|
| hPP | | | |
| Construct 1 | 5.4e-11 | 1.8e-12 | 3 |
| Construct 2 | 5.1e-11 | 4.5e-12 | 3 |
| Construct 3 | 5.1e-11 | 1.6e-12 | 3 |
| hPYY | | | |
| Construct 1 | 2.3e-9 | 4.3e-11 | 3 |
| Construct 2 | 1.9e-8 | 6.6e-9 | 6 |
| Construct 3 | 2.3e-9 | 2.8e-10 | 3 |
| hNPY | | | |
| Construct 1 | 2.1e-7 | 4.6e-8 | 3 |
| Construct 2 | 1.6e-7 | 2.5e-8 | 3 |
| Construct 3 | 2.0e-7 | 1.5e-9 | 3 |

Construct I wildtype
Construct 2 C718T = R240C (in IL3)
Construct 3 G826A = V276M (in TM6)

Transfections into CHO cells were performed for stable expression. Approximately 500 ng of each linearized plasmid was transfected.

### Selection of transfected CHO cells for stable expression initiated with G418.

### Evaluation of cAMP experiments.

Results showed that wild type receptor (after stimulation with forskolin) responded to hPP with 30% reduction of cAMP production. Construct 2 and 3 showed no response to the binding stimulus anymore.

### REFERENCES

Hager J, Dina C, Francke S, et al. (1998) A genome-wide scan for human obesity genes reveals a major susceptibility locus on chromosome 10. Nat Genet, 20: 304-8.
Hebebrand J, Hescker H, Himmelmann GW, Schäfer H, Remschmidt H (1994) Percentiles for the body mass index based on data of the German national nutrition survey and a review of relevant factors with an influence on body weight. Aktuelle Ernahrungsmedizin 19: 259-265.
Hebebrand J, Himmelmann GW, Hescker H, Schäfer H, Remschmidt H (1996) Use of percentiles for the body mass index in anorexia nervosa : diagnostic, epidemiological and therapeutic considerations. Int J Eat Dis 19: 359-369.
Hebebrand J, Hinney A, Roth H et al. (1998) Genetische Aspekte der Adipositas. In:J Wechsler (ed) Adipositas. Ex Libris Roche. Blackwell Verlag : 105-118.
Hinney A, Schmidt A, Nottebom K, et al. (1999) Several mutations in the melanocortin-4 receptor gene including a nonsense and a frameshift mutation associated with dominantly inherited obesity in humans. J. Clin Endocrinol Metab. Apr; 84(4):1483-6.
Hinney A, Ziegler A, Oeffiner F, et al (2000) Independent confirmation of a major locus for obesity on chromosome 10. J. Clin Endocrinol Metab. Aug; 85(8):2962-5.
Huszar D, Lynch CA, Fairchild-Huntress V, et al. (1997) Targeted disruption of the melanocortin-4 receptor results in obesity in mice. Cel 88:131-41.
Price RA, Li WD, Bernstein A, et al. (2001) A locus affecting obesity in human chromosome region 10p12. Diabetologia. Mar;44(3):363-6.
Rankinen T, Perusse L, Weisnagel SJ, et al. (2002) The human obesity gene map: the 2001 update. Obes Res. Mar;10(3):196-243.
Schneider R (1996) Relevanz und Kosten der Adipositas in Deutschland. Ernährungs-Umschau 43:369-374.
Vaisse C, Clément K, Guy-Grand B et al (1998) A frameshift mutation in human MC4R is associated with a dominant form of obesity. Nat Genet 20:113-4.
WHO (1998) Preventing and managing the global epidemic. WHO, Geneva.
Yeo GS, Farooqi IS, Aminian S, et al (1998) A frameshift mutation in MC4R associated with dominantly inherited human obesity. Nat Genet 20:111-2.
Wolf AM, Colditz GA (1996) Social and economic effects of body weight in the United States. Am J Clin Nutr 63(3 Suppl):466S-469S.
Zhang Y, Proenca R, Maffei M, Barone M, Leopold L, Friedman JM. (1994) Positional cloning of the mouse obese gene and its human homologue. Nature 372: 425-432.

## Claims

1. A method of detecting the presence of or predisposition to obesity in a subject, the method comprising (i) providing a sample from the subject and (ii) detecting the presence of an alteration in the PPYR1 gene locus in said sample, said alteration being SNP 718 (nucleotide 803 C/T in SEQ ID N°1).

2. The method according to claim 1, wherein said alteration is detected through the genotyping of an haplotype, said haplotype comprising SNP 718 (nucleotide 803 C/T in SEQ ID N°1), SNP 826 (nucleotide 911 G/A in SEQ ID N°1) and a SNP selected from the group consisting of SNP 195 (nucleotide 280 G/A in SEQ ID N°1), SNP 588 (nucleotide 673 G/A in SEQ ID N°1), SNP 897 (nucleotide 982 C/T in SEQ ID N°1), SNP 948 (nucleotide 1033 C/T in SEQ ID N°1) and SNP 1047 (nucleotide 1132 C/T in SEQ ID N°1).

3. The method according to any one of claims 1-2, wherein the presence of an alteration in the PPYR1 gene locus is detected by sequencing, selective hybridisation and/or selective amplification.

4. The method according to claim 3, wherein said method comprises specifically amplifying PPYR1a gene, preferably with the amplification primer FN1A (SEQ ID No 9).

5. The method of claim 1, comprising detecting the presence of an altered PPYR1 polypeptide, that comprises aminoacid polymorphism 240:Arg/Cys.

6. The method of claim 5, comprising contacting the sample with an antibody specific for said altered PPYR1 polypeptide and determining the formation of an immune complex.

## Patentansprüche

1. Ein Verfahren zum Nachweisen des Vorhandenseins von oder der Prädisposition für Adipositas in einem Patienten, wobei das Verfahren die Schritte umfasst (i) das Bereitstellen einer Probe von dem Patienten und (ii) das Nachweisen des Vorhandenseins einer Veränderung im PPYR1-Genlocus in der Probe, wobei die Veränderung SNP 718 (Nukleotid 803 C/T in SEQ ID NO:1) ist.

2. Das Verfahren nach Anspruch 1, wobei die Veränderung nachgewiesen wird durch Genotypisierung eines Haplotyps, wobei der Haplotyp umfasst: SNP 718 (Nukleotid 803 C/T in SEQ ID NO:1), SNP 826 (Nukleotid 911 G/A in SEQ ID NO:1) und ein SNP ausgewählt aus der Gruppe bestehend aus SNP 195 (Nukleotid 280 G/A in SEQ ID NO:1), SNP 588 (Nukleotid 673 G/A in SEQ ID NO:1), SNP 897 (Nukleotid 982 C/T in SEQ ID NO:1), SNP 948 (Nukleotid 1033 C/T in SEQ ID NO:1), und SNP 1047 (Nukleotid 1132 C/T in SEQ ID NO:1).

3. Das Verfahren nach einem der Ansprüche 1 bis 2, wobei das Vorhandensein einer Veränderung im PPYR1-Genlocus durch Sequenzierung, selektive Hybridisierung und/oder durch selektive Amplifikation nachgewiesen wird.

4. Das Verfahren nach Anspruch 3, wobei das Verfahren das spezifische Amplifizieren des PPYR1a-Gens umfasst, vorzugsweise mit dem Amplifikations-Primer FN1A (SEQ ID NO:9).

5. Das Verfahren nach Anspruch 1, wobei das Verfahren das Nachweisen des Vorhandenseins eines veränderten PPYR1-Polypeptids umfasst, das den Aminosäure-Polymorphismus 240:Arg/Cys umfasst.

6. Das Verfahren nach Anspruch 5, wobei das Verfahren das Zusammenbringen der Probe mit einem Antikörper, der spezifisch für das PPYR1-Polypeptid ist, und das Bestimmen der Bildung eines Immunkomplexes umfasst.

## Revendications

1. Procédé de détection de la présence ou de la prédisposition à l'obésité chez un sujet, le procédé comprenant (i) la fourniture d'un échantillon du sujet (ii) la détection de la présence d'une altération dans le locus du gène PPYR1 dans ledit échantillon, ladite altération étant SNP 718 (nucléotide 803 C/T dans SEQ ID N°1).

2. Procédé selon la revendication 1, dans lequel ladite altération est détectée par génotypage d'un haplotype, ledit haplotype comprenant le SNP 718 (nucléotide 803 C/T dans SEQ ID N°1), SNP 826 (nucléotide 911 G/A dans SEQ ID N°1) et un SNP sélectionné dans le groupe composé de SNP 195 (nucléotide 280 G/A dans SEQ ID N°1), SNP 588 (nucléotide 673 G/A dans SEQ ID N°1), SNP 897 (nucléotide 982 C/T dans SEQ ID N°1), SNP 948 (nucléotide 1033 C/T dans SEQ ID N°1) et SNP 1047 (nucléotide 1132 C/T dans SEQ ID N°1).

3. Procédé selon l'une quelconque des revendications 1 et 2, dans lequel la présence d'une altération dans le locus du gène PPYR1 est détectée par séquençage, hybridation sélective et/ou amplification sélective.

4. Procédé selon la revendication 3, dans lequel ledit procédé comprend le gène PPYR1a spécifiquement amplifié, de préférence avec l'amorce d'amplification FN1A (SEQ ID No9).

5. Procédé selon la revendication 1, comprenant la détection de la présence d'un polypeptide PPYR1 altéré qui comprend le polymorphisme de l'acide aminé 240:Arg/Cys .

6. Procédé selon la revendication 5, comprenant la mise en contact de l'échantillon avec un anticorps spécifique dudit polypeptide altéré PPYR1 et la détermination de la formation d'un complexe immun.
